(19) Europäisches Patentamt
European Patent Office
Office européen des brevets

(11) Publication number: **0 109 273**
**B1**

(12) # EUROPEAN PATENT SPECIFICATION

(45) Date of publication of patent specification: **03.02.88**

(51) Int. Cl.$^4$: **C 07 D 301/12**, B 01 J 31/02

(21) Application number: **83306883.6**

(22) Date of filing: **10.11.83**

(54) **Peroxide compositions based on tungsten and phosphorus or arsenic, and a process for preparing the same.**

(30) Priority: **10.11.82 IT 2415482**

(43) Date of publication of application:
**23.05.84 Bulletin 84/21**

(45) Publication of the grant of the patent:
**03.02.88 Bulletin 88/05**

(84) Designated Contracting States:
**BE CH DE FR GB LI NL**

(56) References cited:
**EP-A-0 057 533**
**FR-A-1 506 459**
**FR-A-2 082 811**
**GB-A-2 055 821**
**US-A-3 766 232**

**CHEMICAL ABSTRACTS, vol. 74, no. 2, 11th January 1971, page 230, no. 6813s, Columbus, Ohio, US**

(73) Proprietor: **Montedison S.p.A.**
**31, Foro Buonaparte**
**I-20121 Milan (IT)**

(72) Inventor: **Venturello, Carlo**
**135, Via XXIII Marzo**
**Novara (IT)**
Inventor: **D'Aloisio, Rino**
**25, Via Romentino**
**Novara (IT)**
Inventor: **Ricci, Marco**
**5, Via Brescia**
**Novara (IT)**

(74) Representative: **Whalley, Kevin et al**
**MARKS & CLERK 57/60 Lincoln's Inn Fields**
**London WC2A 3LS (GB)**

**EP 0 109 273 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to peroxide compositions based on tungsten and phosphorus or arsenic, more particularly to peroxidic compositions based on tungsten chemically associated with phosphorus or arsenic and with quaternary onium cations.

The invention relates moreover to a process for preparing such peroxide compositions, and to their use especially as epoxidation catalysts.

GB—A—2055821 discloses a process for the catalytic epoxidization of olefins by reaction with hydrogen peroxide according to the double phase technique with onium salts, wherein the reaction is conducted in a liquid aqueous-organic two-phase system consisting of:

a) an organic phase substantially containing the olefin and

b) an aqueous acidic phase substantially containing the hydrogen peroxide, in the presence of a catalytic system consisting of at least one element or a derivative thereof selected from W, Mo, V and of at least one derivative of P or As.

EP—A—57533 discloses catalysts useful for the reaction of a tertiary olefin containing hydrocarbon mixture with other compounds which consist of two components, such as phosphorus and tungsten in the form of heteropoly acids. This reaction can be carried out on supports as carriers such as silica, active carbon or alumina.

The present invention in one aspect provides a peroxidic composition, characterized by substantially consisting of a peroxy-phospho-tungstate and/or a peroxy-arseno-tungstate having the following formula (I):

$$Q_3XW_4O_{24-2n} \qquad (I)$$

wherein X represents a P or As atom; $n$ is 0, 1 or 2; and Q represents a cation of a quaternary onium salt having the formula (II):

$$(R_1, R_2, R_3, R_4\ M)^+Y^- \qquad (II)$$

wherein M represents a pentavalent element of Group VA of the Periodic System; $Y^-$ represents an inorganic anion; and $R_1$, $R_2$, $R_3$ and $R_4$, which may be the same as or different from each other, each represent a hydrogen atom or a monovalent hydrocarbon group having up to a total of 70 carbon atoms.

Such compositions may be either in solid crystalline form or in the form of viscous oils.

The compositions of the invention are obtained starting from a suitable oxygenated tungsten derivative and from a suitable phosphorus or arsenic oxygenated derivative and hydrogen peroxide, contained in an acid aqueous phase maintained at a pH value below 2, by reaction with a quaternary onium salt (as defined above) or a precursor thereof, according to another aspect of the present invention.

The compositions of the above defined formula (I) are considered to be structurally formed by an anionic complex that comprises tungsten with phosphorus or arsenic, and by quaternary onium cations as defined above.

Nonetheless, the compositions of the present invention may be defined as the products resulting from the reaction of the abovementioned starting compounds, under operational conditions that will be indicated subsequently, and thus the above suggested structure must be understood as being a reasonable supposition within the scope of formula (I).

The compositions thus obtained, whether oily liquid or solid, represent useful compounds having particular applications. In fact, due to the active oxygen present, these compounds may be used as oxidizing agents in general, and especially in epoxidation reactions of compounds having olefinic bonds, with respect to which compounds they show a considerable epoxidizing activity.

More particularly, when they are used as reactants the compositions of this invention make it possible to prepare epoxides starting from olefines; or, used in small quantities, they represent excellent catalysts in a homogeneous phase or according to the phase-transfer technique, for the catalystic epoxidation of unsaturated compounds carried out with the help of oxidizing agents, such as hydrogen peroxide.

In this connection, the compositions of formula (I) as previously defined, may be represented also by the following equivalent formula:

$$Q_3XW_4O_{16}(O_{act})2m$$

in which $m$ is 2, 3 or 4, while index 'act' stands for the word 'active'.

The epoxidized (=epoxy) compounds thus obtained, such as the epoxides (=epoxy compounds) of olefines, are chemical products of a considerable economic importance, which find applications in industry, even on a large scale.

In fact, in addition to being useful intermediates for organic syntheses in general, amongst other possible applications are intermediates in the production of urethanes, in the production of foamed materials, of glycols for lubricants, of surfactants, of esters for plasticizers, and of polyester resins. Lastly, the epoxides may find direct application in the preparation of thermosetting epoxy resins.

Organo-metal complexes of molybdenum and of tungsten have been described as catalysts suited for the epoxidation of olefinic bonds with hydrogen peroxide, for example molybdenum or tungsten complexes with amides of carboxylic acids or mineral acids; or containing organic binders, which binders are pyridine oxides or heterocyclic nitrogen compounds such as hydroxy-quinolin or picolinic acid.

Nevertheless, apart from the chemical diversity of the above mentioned organo-metal complexes of W or Mo, with respect to the compositions of formula (I) of the present invention, their activity as catalysts in general is found to be limited to homogeneous catalysis in a single reaction phase containing $H_2O$, $H_2O_2$, catalyst and olefine, dissolved in a homogeneous organic solvent medium common to all.

In fact, the olefine is in general insoluble in an aqueous phase and thus there is used $H_2O_2$ in a high concentration (greater than 70%).

These preceding operational conditions entail extremely slow reaction speeds, a low productivity of the oxidized product (epoxides, etc.) because of the limited solubility of $H_2O_2$ in the homogeneous solvent/olefine/catalyst system, or low conversions and low selectivity for the presence of hydrolysis products.

Due to their characteristics of good solubility in the standard organic solvents and, in general, low or insignificant solubility in water, the compositions of formula (I) of the present invention are especially suited for use as epoxidation catalysts according to the phase-transfer technique.

The present invention thus provides a class of tungsten and phosphorus or arsenic-based compositions, containing active oxygen and quaternary onium cations, having useful application in oxidation reactions, and especially in the epoxidation of compounds with olefinic bonds, preferably carried out according to the so-called phase-transfer technique.

As previously described, the class of compositions of formula (I) as herein above defined are prepared by a process wherein a suitable oxygenated tungsten derivative, a suitable oxygenated derivative of P or As and hydrogen peroxide, contained in an aqueous acid phase maintained at a pH value below 2, are made to react with a quaternary onium salt (as defined above) or with a precursor thereof, possibly contained in an organic phase immiscible with the aqueous phase.

From the reaction mass, the composition is isolated according to conventional methods, for example by separation of the organic phase, filtration of the organic phase and evaporation of the filtrate.

More particularly, the compositions of the present invention may be prepared by the reaction between a suitable oxygenated tungsten compound, a suitable oxygenated phosphorus or arsenic compound and hydrogen peroxide, all contained in an acid aqueous phase maintained at a pH value below 2, with a said quaternary onium salt, possibly contained in an organic, substantially water immiscible solvent, according to pre-established, even if not critical, molar ratios, generally under conditions of substantially atmospheric pressure and at a temperature preferably comprised from 20°C to 80°C, but in any case from 0°C to 100°C.

In the preparation of the compositions of formula (I) there are suitably used oxygenated W (VI) compounds such as tungstic acid or the corresponding salts of alkaline metals.

In general, however, there may be used any derivative of tungsten envisaged or tungsten itself, which derivative or metal, under the envisaged reaction conditions, may form *in situ* the oxygenated W (VI) derivative. Thus, there may be used for example: $WO_2$, $W_2O_5$, $WO_3$, $WS_2$, $WS_3$, W oxychloride, W chloride, or W-hexacarbonyl.

Analogously, in the reaction there are suitably used oxygenated compounds of P (V) or of As (V), such as phosphoric acid, arsenic acid and their alkaline salts.

Also in this case, however, there may be used any one of the P or As derivatives, which derivative may, under the envisaged reaction conditions, form *in situ* the phosphate or arsenate ion.

There may thus be used for example: $P_2O_5$, $As_2O_5$, $PCl_5$, $AsCl_5$, $POCl_3$, $AsOCl_3$, and polyphosphoric acid and its alkaline salts. There may also be used water-soluble salts containing As and W, or P and W, for example those of arsenotungstic and phosphotungstic acids.

The onium salts consist of known quaternary salts of formula (II):

$$(R_1, R_2, R_3, R_4M)^+Y^- \qquad (II)$$

wherein:

M represents a pentavalent element belonging to Group VA of the Periodic System;

$Y^-$ represents a stable inorganic anion such as $Cl^-$, $HSO_4^-$, or $NO_3^-$;

$R_1$, $R_2$, $R_3$ and $R_4$, which may be the same as or different from each other, each represent hydrogen or a monovalent hydrocarbon group having a total number of carbon atoms of up to 70, but preferably from 25 to 40, for reasons which will be described in more detail subsequently.

Depending on whether M is either an N, P, As or Sb atom, there will be obtained the corresponding onium salts, i.e. ammonium (N), phosphonium (P), arsonium (As), stibonium (Sb).

As an onium salt source there may, moreover, be used onium salts, preferably chlorides, immobilized on macroporous polymeric matrices of the polystyrenic or silicone type, which matrices are preparable according to known techniques or are commercially available.

The compositions obtained with the onium salts fixed on polymeric matrices are useful as epoxidation catalysts for olefines.

Thus, according to another aspect of the present invention, there is provided a process for the

preparation of an epoxidation catalyst fixed on a polymeric macroporous polystyrenic or silicone resin, wherein an oxygenated tungsten derivative, and an oxygenated derivative of P or As and hydrogen peroxide, contained in an aqueous acid phase having a pH below 2, is reacted with an onium salt fixed on a polymeric macroporous polystyrenic or silicone resin.

In this preparation process the pH of the aqueous phase is maintained below 2, but usually not below zero.

The exact structure of these catalysts fixed on resins has not been established. For simplicity's sake, in the following they will be indicated as "catalysts fixed on resins".

The catalysts fixed on resins are insoluble in aqueous and organic solvents and, thus, prove to be particularly effective in the triple organic liquid/aqueous liquid/solid phase. They prove particularly interesting because of the possibility of their recovery at the end of the reaction, due to their insolubility.

Lastly, in the process for the preparation of the compositions of formula (I), the ammonium quaternary salts, for M=N in formula (II), as herein above defined, may be substituted by the equivalent primary, secondary and tertiary amines, which in the existing reaction system may give place to quaternized species.

As organic solvents for the onium salt there are used, in general, in the preparation process of compositions of the formula (I), inert solvents substantially immiscible with the aqueous phase containing the W/P or W/As compound, and capable of solubilizing the reaction product. Particularly suitable for this purpose are aromatic hydrocarbons such as benzene, toluene, and xylenes, and chlorinated hydrocarbons such as dichloromethane, dichloroethane, trichloroethane, and chlorobenzene, and mixtures of such solvents.

In the preparation of the compositions of formula (I), the pH value of the aqueous phase, containing hydrogen peroxide and the soluble oxygenated W- and P- or As derivatives, is maintained below 2. Usually, the pH is not lower than zero. The pH value may be adjusted if required by means of mineral acids.

The molar ratios of the reactants are not critical with respect to the formation of the compositions of formula (I), but the following ratios have been found to ensure advantageous results as far as yield and purity of the product are concerned. There are thus preferably used, for each mol of P or As compound, expressed as P or As, at least 4 mols of W compound, expressed as W, and up to 2 mols of onium salt. These values are optimal values, and while greater quantities of P or As compound do not bring any advantage, greater quantities of onium salts cause a gradual drop in the purity.

There are preferably used from 2.5 to 6 mols of $H_2O_2$ per mol of W (VI) compound. Greater values are compatible though not advantageous. When there are used W compounds with a valency below VI, to the said quantity of $H_2O_2$ there must also be added the quantity necessary for bringing the W to the state of oxidation VI.

The concentration of the reactants in the aqueous as well as in the organic phase does not represent a critical parameter, nor does the reaction time.

The compositions of formula (I) according to the present invention are in the physical state either crystallizable solids or thick oily liquids.

In general the compositions of formula (I) are soluble in conventional organic solvents such as alcohols, ketones, chlorinated hydrocarbons, and aromatic hydrocarbons, such as methyl alcohol, ethyl alcohol, acetone, ethylmethylketone, methylene chloride, dichloroethane, benzene, toluene, and xylenes.

On the contrary, compositions of formula (I) are barely soluble or are insoluble in water, in direct function of the number of carbon atoms and/or of the nature of the radicals from $R_1$ to $R_4$ of the onium salt used; the solubility in water rises considerably in the case of salts with a low total number of carbon atoms, of the order of 20 carbon atoms.

Lastly, the compositions prove to have active oxygen which makes them particularly suited for the applications previously mentioned.

The compositions of formula (I) may be suitably obtained in the following way.

The oxygenated W (VI) derivative (for example tungstic acid) and the oxygenated derivative of P (V) or of As (V) (for example phosphoric acid or arsenic acid), in chosen molar ratios, either in an aqueous suspension or solution having a pH value (possibly corrected with a mineral acid) below 2, are treated, under stirring, with an aqueous $H_2O_2$ solution in the desired ratio, at a temperature from 20°C to 80°C.

Then, under stirring, there is admixed, preferably at room temperature, the pre-established quantity of onium salt dissolved in an organic solvent (dichloroethane, benzene) immiscible with water.

The resulting biphasic mixture is kept under stirring for between 15 and 30 minutes. If the product thus formed appears in the solid state, it will be directly separated from the biphasic mixture by filtering, etc. If the product is in the liquid state, the organic phase will be separated and filtered and then evaporated under vacuum at a temperature of 40°—50°C, thereby obtaining the composition of formula (I) in the form of either a solid or of a thick oil.

Alternatively, in the presence of onium salts particularly soluble in $H_2O$, it is possible to operate in a single aqueous phase. In this case the separation of the product is achieved by filtering (if solid) or by extraction with solvents of the aqueous phase, etc. (if oily).

In their turn, the catalysts fixed on resins are obtained by treating in an acid aqueous phase the compound of W (e.g. tungstic acid) and of As (e.g. arsenic acid) or of P (e.g. phosphoric acid), with $H_2O_2$, as previously above described at a temperature preferably from 20° to 80°C. Thereupon there is added the

organic solvent (e.g. toluene) and the onium salt on a polymer, preferably in the form of chloride, and stirring is continued for about 2 hours at a temperature from 60° to 100°C. The catalyst fixed on resin is then separated, e.g. by filtering.

As indicated previously, the compositions of formula (I) and the catalysts fixed on resins, of the present invention, when used as epoxidation catalysts for olefines, may be used according to the phase transfer catalysis aqueous liquid/organic liquid with catalyst (I) or aqueous liquid/organic liquid/solid, with the catalyst fixed on the resin.

Compositions of formula (I) may, furthermore, be supported, in a conventional way, on porous, inert solid materials such as clays, bauxites, Kieselgur, alumina, pumice, and zeolites.

The supporting is achieved according to conventional methods by successive impregnations of the support with solutions containing the composition of formula (I), according to composition (I)/support ratios (catalyst/carrier ratios) within a wide range, i.e. for example from about 0.01:1 to about 0.05:1 by weight.

The oxidization reactions in which it is possible to use compositions of formula (I) either as oxidizing agents or as catalysts, or the catalysts fixed on resins, as catalysts, include the epoxidation of olefines as indicated above, and also the oxidization of sulphides to sulphoxides.

More particularly, the composition of formula (I) and the catalysts fixed on resins, as indicated above, find their most effective application as epoxidation catalysts for olefinic compounds with $H_2O_2$ as oxidizing agent, according to the aqueous liquid/organic liquid or the aqueous liquid/organic liquid/solid or the aqueous-organic liquid/solid technique.

In the case of the epoxidation reaction of olefines with $H_2O_2$ catalyzed by the compositions of formula (I), there is utilized a phase-transfer system, in general aqueous liquid/organic liquid, essentially consisting of:

(a) an organic phase substantially containing catalyst (I), of the present invention, the olefine to be epoxidized, and the possible solvent; and

(b) an aqueous phase containing substantially the $H_2O_2$.

Alternatively, the catalyst fixed on resin or catalyst (I) on a support may form a third solid phase (c).

The use of compositions of formula (I) and of the catalysts fixed on resins, according to the present invention, as catalysts according to the above indicated technique, allows the $H_2O_2$ to be used in a much diluted form, even at a titre below 10%, and to obtain at the same time a high conversion rate of the $H_2O_2$ itself, combined with a high selectivity in the epoxide of the olefine, without the necessity, met in processes of the prior art, to homogenize the system with the use of suitable solvents and, above all, without having to carry out burdensome operations for the removal of water from the reaction medium.

Thus, the compositions of formula (I) and the catalysts fixed on resins of the present invention, when applied in reactions of this type, on the one hand ensure results superior to those obtainable with the best catalytic systems based on organo-metal compounds with active oxygen, acting in a homogeneous or heterogeneous phase, and on the other hand unlike these latter they are found to be exceptionally suited for being used according to the above said phase transfer technique, obtaining economical and operational advantages.

The epoxidation reaction of the olefines, using as catalysts compositions of formula (I) and the catalysts fixed on resins of the present invention, occurs preferably under the following operational conditions.

The epoxidation reaction, which may be represented by the following scheme:

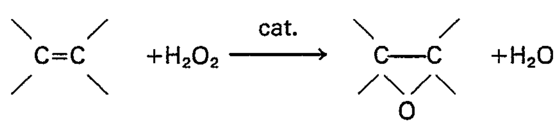


is conducted in a biphasic aqueous/organic system, under vigorous stirring in the presence of catalyst (I) of the present invention.

The organic phase contains the olefine and a possible organic solvent while the aqueous phase contains the hydrogen peroxide.

In the case of the use of the catalyst fixed on resin, the reaction is conducted under the same operational conditions, in such a case in the presence of a triphasic aqueous liquid/organic liquid/solid system as indicated above.

The operational temperature and pressure are practically determined by the reactivity and by the nature of the olefine and by the stability of the hydrogen peroxide and of the catalyst used.

Temperatures from 0° to 120°C, and pressures from atmospheric pressure to 100 atmospheres may be regarded as operationally sufficient.

The olefines which may be subjected to the epoxidation reaction may be of the following formula:

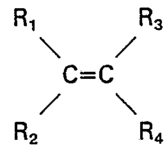

wherein $R_1$, $R_2$, $R_3$ and $R_4$, optionally substituted with functional groups inert under reaction conditions, may be the same as or different from each other and represent hydrogen atoms or hydrocarbon groups, such as alkyls and alkenyls, having up to 30 carbon atoms, cycloalkyls and cycloalkenyls having from 3 to 12 carbon atoms and optionally branched, and aryls, alkyl-aryls and alkenyl-aryls having from 6 to 12 carbon atoms; moreover an $R_1$, $R_2$, $R_3$ or $R_4$ group, taken together with an adjacent group, may represent an alkyl or alkenyl group having up to 12 carbon atoms in the resulting cycle.

Substituent groups, inert under reaction conditions, are, for example, the hydroxy, halogen (Cl, Br, F, I), nitro, alkoxy, amine, carbonyl, carboxylic, ester, amide, and nitrile groups.

As indicated above, groups $R_1$, $R_2$, $R_3$ and $R_4$ may also be alkenyls; in other words, the process according to this invention is applicable also to polyolefines such as dienes and trienes, whether conjugated or not.

Olefines suitable for the epoxidation according to the present invention, include, for example, unsaturated alkylic, alicyclic, and alkylarylic hydrocarbons such as propylene, butenes, and pentenes, and in general the linear or branched mono- and di-olefines having up to 20 carbon atoms, cyclohexene, norbornene, limonene, camphene, vinylcyclohexene, styrene, alpha-methylstyrene, indene, and stilbene; the unsaturated alkyl halides such as allyl halides; unsaturated acids and their esters, such as acrylic, methacrylic, crotonic, and oleic acids; unsaturated alcohols and their esters such as allyl alcohol; and unsaturated aldehydes and unsaturated ketones.

The duration of the reaction depends on the nature of the catalyst and on the type of solvent and of olefine used; in general, duration times comprised between just a few minutes and a few hours are quite sufficient for completing the reaction.

The catalyst is generally used in quantities from 0.0001 to 1 g/atom of metal per 1 mol of hydrogen peroxide, more preferably from about 0.005 to about 0.05 g/atom per 1 mol.

There may furthermore be used mixtures of compositions of formula (I). Mixtures of this type may be obtained, for example, using commercial onium salt mixtures.

As already indicated above, the reaction is conducted under phase-transfer conditions, for example in a bi-phasic aqueous liquid/organic liquid system.

More particularly, organic phase (a) may be optionally constituted of the same reacting olefine used in a suitable excess, or it may be constituted of the reacting olefine dissolved in organic solvent.

As solvents for the organic phase there are used inert solvents, substantially immiscible with the aqueous phase; effective practical results are obtained by using aromatic hydrocarbons such as benzene, toluene, xylenes, chlorinated hydrocarbons such as dichloromethane, trichloromethane, chloroethane, chloropropane, dichloroethanes, trichloroethanes, tetrachloroethanes, di- and trichloropropanes, tetrachloropropanes, chlorobenzene, alkyl esters such as ethyl acetate or suitable mixtures thereof.

The choice of the type of organic phase (a) in each instance depends on the reactivity of the starting olefine and on the parameters used for the reaction.

In the case in which in the organic phase there are used the above described inert solvents, the concentration of the olefine in the solvent is not critical with regard to the carrying out of the process.

Preferred concentrations of the olefine in the organic phase and from about 5% to about 95% by weight, although both higher values or lower values are compatible within the limits of their practability.

The concentration of the hydrogen peroxide in the aqueous phase may be suitably maintained from about 0.1% to about 70%.

Nonetheless, the epoxidation reaction, conducted by the use of the catalysts of this invention, offers the advantage of allowing operation with low concentration values for the hydrogen peroxide. Effective values of the hydrogen peroxide concentration are from 1% to about 10%; however, lower values are still possible. This is economically favourable in comparison with the costly preparation of the solutions with concentrations higher than 70% used in the prior art, and the problems of operational safety due to the necessity to maintain high concentration throughout the course of the process.

The invention will be further described with reference to the following illustrative examples.

In the Examples, the yields have been calculated with reference to the quantity of onium salt used in the preparation of compositions of formula (I) and of catalysts fixed on resins; w/v stands for weight/volume.

Example 1

Into a beaker were placed:

2.50 grams of $H_2WO_4$ (10 mmols),

7 ml of $H_2O_2$ in a 30% concentration w/v (300 g/liter) (about 62 mmols).

The tungstic acid suspension was maintained under stirring at about 60°C until complete dissolution of the tungstic acid occurred.

After cooling down, the colourless resulting solution was additioned with 0.62 ml of a 40% w/v $H_3PO_4$ (400 g/liter) (2.5 mols).

The solution thus obtained was diluted with $H_2O$ to 30 ml, then filtered and introduced into a reactor fitted with a dripper and a stirrer. Under stirring, there were then dripped into the reactor, in about 2 min, 1.60 g of methyltrioctylammonium chloride (about 4 mmols) dissolved in 40 ml of methylene chloride.

After 15 minutes of further stirring, the organic phase was separated, filtered and evaporated under vacuum at from 40°C to 50°C.

Thereby there were obtained 2.82 g (95.9% with respect to the onium salt used) of a thick, colourless oil which, according to a percentual analysis, was found to be of the formula:

$$C_{75}H_{162}N_3PW_4O_{22}=[(C_8H_{17})_3N\ CH_3]_3PW_4O_{22}$$

| Elementary analysis | Theoretical % | Found % |
|---|---|---|
| C | 40.49 | 40.35 |
| H | 7.34 | 7.42 |
| N | 1.89 | 1.85 |
| P | 1.39 | 1.32 |
| W | 33.06 | 32.79 |

Active [0] found (determined by iodometry in acetic acid)=4.33%; active [0] theoretical (calculated for 6 $O_{act}$)=4.315%. Molecular weight (in $CHCl_3$)=2190 (theoretical=2224.7).

Example 2

There was followed the procedure described in Example 1, but substituting the methyltrioctylammonium chloride with 1.56 g of tetrahexylammonium chloride (about 4 mmols) dissolved in 40 ml of benzene. From the biphasic mixture was then directly separated a white solid which was filtered, washed with $H_2O$, then washed with a little benzene and finally dried on a porous plate. Thereby there were obtained 2.35 g (80.8%) of a product which, according to a percentual analysis, was found to be of the formula:

$$C_{72}H_{156}N_3PW_4O_{22}=[(C_6H_{13})_4N]_3PW_4O_{22}$$

| Elementary analysis | Theoretical % | Found % |
|---|---|---|
| C | 39.62 | 39.17 |
| H | 7.20 | 7.19 |
| N | 1.92 | 1.90 |
| P | 1.42 | 1.44 |
| W | 33.70 | 33.57 |

Active [0] found=4.42%; active [0] theoretical (calculated for 6 $O_{act}$)=4.40%. Molecular weight (in $CHCl_3$)=2210 (theoretical=2182.6).

Example 3

There was followed the procedure described in Example 2, but substituting the $H_3PO_4$ with 0.78 g of Na $HAsO_4 \cdot 7H_2O$ (2.5 mmols) dissolved in 3—4 cc of $H_2O$ and acidified with 3.5 ml of $H_2SO_4$ at a 30% concentration. From this biphasic mixture was thereupon directly separated a white solid which was filtered, washed first with $H_2O$ and then with a little benzene, and finally dried on a porous plate. Thereby there were obtained 2.4 g (79.7%) of a product which, according to a percentual analysis, was found to be of the formula:

$C_{72}H_{156}N_3AsW_4O_{24}=[(C_6H_{13})_4N]_3AsW_4O_{24}$

| Elementary analysis | Theoretical % | Found % |
|---|---|---|
| C | 38.29 | 38.36 |
| H | 6.96 | 6.97 |
| N | 1.86 | 1.89 |
| As | 3.32 | 3.24 |
| W | 32.57 | 32.57 |

Active [0] found=5.61%; active [0] theoretical (calculated for 8 $O_{act}$)=5.67%. Molecular weight (in $CHCl_3$)=2200 (theoretical=2258.6).

Example 4

There was followed the procedure described in Example 1, but substituting the methyltrioctylammonium chloride with 1.36 g of tetrabutylammonium bisulfate (about 4 mmols) dissolved in 15 ml of $H_2O$ instead of in an organic solvent immiscible with water, thus operating in a single aqueous phase.

A precipitated white solid was thereupon filtered, washed with a little $H_2O$ (10 ml) and then dried on a porous plate. Thereby there were obtained 2.2 g (87.8%) of product, which was found to be partially soluble in $H_2O$ and very soluble in conventional solvents (dichloroethane, acetone, $CH_2Cl_2$, etc.) and which, according to a percentual analysis, was found to be of the following formula:

$C_{48}H_{108}N_3PW_4O_{24}=[(C_4H_9)_4N]_3PW_4O_{24}$

| Elementary analysis | Theoretical % | Found % |
|---|---|---|
| C | 30.70 | 30.74 |
| H | 5.80 | 5.82 |
| N | 2.24 | 2.23 |
| P | 1.65 | 1.59 |
| W | 39.17 | 39.34 |

Active [0] found=6.76%; active [0] theoretical (calculated for 8 $O_{act}$)=6.815%. Molecular weight (in $CH_2Cl_2$)=1930 (theoretical=1878).

Example 5

There was followed the procedure described in Example 1, but substituting the $H_3PO_4$ with 0.78 g of $Na_2HAsO_4$, $7H_2O$ (2.5 mmols) dissolved in 3—4 ml of $H_2O$ and acidified with 3.5 ml of $H_2SO_4$ in a 30% concentration. Thereby there were obtained 2.76 g (93.5%) of a waxy product which, according to a percentual analysis, was found to be of the formula:

$C_{75}H_{162}N_3AsW_4O_{20}=[(C_8H_{17})_3NCH_3]_3AsW_4O_{20}$

| Elementary analysis | Theoretical % | Found % |
|---|---|---|
| C | 40.27 | 40.47 |
| H | 7.30 | 7.32 |
| N | 1.88 | 1.89 |
| As | 3.35 | 3.40 |
| W | 32.69 | 32.60 |

Active [0] found=2.75%; active [0] theoretical (calculated for 4 $O_{act}$)=2.86%. Molecular weight (in $CHCl_3$)=2225 (theoretical=2236.6).

Example 6

There was followed the procedure described in Example 4, but substituting the $H_3PO_4$ with 0.78 g of $Na_2HAsO_4 . 7H_2O$ (2.5 mols) dissolved in 3—4 ml of $H_2O$ and acidified with 3.5 ml of $H_2SO_4$ in a 30% concentration. Thereby there were obtained 2.18 g (84.8%) of a white solid which, according to a percentual analysis, was found to be of the formula:

$C_{48}H_{108}N_3AsW_4O_{24}=[(C_4H_9)_4N]_3AsW_4O_{24}$

| Elementary analysis | Theoretical % | Found % |
|---|---|---|
| C | 29.9 | 29.67 |
| H | 5.66 | 5.61 |
| N | 2.19 | 2.17 |
| As | 3.90 | 3.85 |
| W | 38.28 | 38.30 |

Active [0] found=6.60%; active [0] theoretical (calculated for 8 $O_{act}$)=6.66%.

Example 7

Into a beaker were placed:

3.30 g of $Na_2WO_4 . 2H_2O$ (10 mmols),

30 ml of $H_2O$,

0.55 g of $NaH_2PO_4 . H_2O$ (4 mmols).

This solution was thereupon acidified with $H_2SO_4$ in a 30% concentration until reaching a pH=1, whereupon there were admixed 3 ml of $H_2O_2$ at a 40% w/v concentration (about 35 mmols).

To the resulting, filtered solution was added tetrahexylammonium chloride (1.56 g), proceeding as in Example 2. Thereby there were obtained 2.30 g (79%) of a white solid which corresponded to the product of Example 2.

Example 8 (catalyst fixed on a resin)

Into a beaker were placed:

14 g of $H_2WO_4$ (56 mmols),

39 ml of $H_2O_2$ in a 30% w/v concentration (344 mmols).

The suspension of tungstic acid was maintained under stirring at about 60°C until full dissolution of the tungstic acid was achieved. After cooling down, the resulting colourless solution was additioned with 3.45 ml of $H_3PO_4$ at a 40% w/v concentration (14 mmols).

The solution was thereupon diluted to 120 ml with $H_2O$ and then filtered. There were then added 40—50 ml of toluene.

Into the mixture thus obtained were then introduced, as an onium salt, 8.75 g of hexyltributylphosphonium chloride supported on a polystyrenic matrix (0.62 milliequivalents of $Cl^-$/1 g of resin) and the whole was then heated under vigorous stirring at 80°C for 2 hours.

The resin was then filtered, washed with little water and then with toluene, after which it was dried on a porous plate. Thereby there were obtained 11.6 g of a resin containing 15.5% of tungsten.

Example 9

Into a 4-necked reactor of 250 ml capacity, fitted with a blade stirrer, a thermometer and a reflux coolant, there were introduced 15 ml of $H_2O$, 10.5 ml of $H_2O_2$ in a 40.14% w/v concentration (corresponding to about 124 mmols), 1.41 g of the composition of Example 1 (corresponding to 2.53 mmols of W) dissolved in 20 ml of 1,2-dichloroethane and 31 ml of 1-octene (about 200 mmols).

The mixture was then quickly brought up to 70°C under vigorous stirring and was then maintained at this temperature for 45 minutes. At the end there were metered by iodometry 1.24 mmols of unreacted $H_2O_2$ in the aqueous phase and by gas chromatography 109.2 mmols of 1,2-epoxyoctane in the organic phase, which corresponded to a conversion of the hydrogen peroxide of 99% with a selectivity in epoxide on the consumed hydrogen peroxide equal to 89%.

Example 10

There was followed the procedure described in 9, but using 1-dodecene (44.3 ml; about 200 mmols) instead of 1-octene. At the end there were metered 1.74 mmols of unreacted $H_2O_2$ (conversion: 98.6%) and 116.6 mmols of 1,2-epoxydodecane (selectivity: 95% calculated on the consumed $H_2O_2$).

Example 11

There was followed the procedure described in Example 9, but using allyl chloride (32.8 ml; about 400 mmols) instead of 1-octene, and benzene (30 ml) instead of 1,2-dichloroethane, and operating at 60°C (temperature of the bath) for 3 hours. At the end there were metered 0.75 mmols of unreacted $H_2O_2$ (conversion: 99.4%) and 99.8 mmols of epichlorohydrin (selectivity: 81% on the $H_2O_2$).

Example 12

There was followed the procedure described in Example 9, using the composition of Example 3 (2 g corresponding to 3.54 mmols of W) instead of the composition of Example 1. At the end there were metered 1.24 mmols of unreacted $H_2O_2$ (conversion: 99%) and 110.5 mmols of 1,2-epoxyoctane (selectivity: 90% on the $H_2O_2$).

Example 13

There was followed the procedure described in Example 1, but substituting the methyltrioctylammonium chloride with 2.30 g of dimethyl [dioctadecyl (75%)—dihexadecyl (25%)] ammonium chloride (Arquad 2HT produced by Akzo Chemie Italia S.p.A.), having an average formula: $C_{37}H_{38}NCl$, (about 4 mmols), dissolved in 40 ml of methylene chloride.

By evaporation of the organic phase (preliminary filtered on paper) at 40°C—50°C under vacuum, there were obtained 3.40 g (93% with respect to the onium salt used) of a white solid which, according to a percentual analysis, was found to be of the following average formula:

$$C_{111}H_{234}N_3PW_4O_{22}=[[C_{18}H_{37}(75\%)+C_{16}H_{33}(25\%)]_2N(CH_3)_2]_3PW_4O_{22}$$

| Elementary analysis | Theoretical % | Found % |
|---|---|---|
| C | 48.84 | 48.79 |
| H | 8.64 | 8.74 |
| N | 1.54 | 1.53 |
| P | 1.135 | 1.15 |
| W | 29.95 | 26.75 |

Active [0] found=3.51%; active [0] theoretical (calculated for 6 $O_{active}$)=3.52%. Average molecular weight (in 1,2-dichloroethane)=2940 (theoretical: 2729.68).

Example 14

There was followed the procedure described in Example 9, using the composition of Example 13 (1171 g, corresponding to 2.5 mmols of W) instead of that of Example 1, using benzene (20 ml) instead of 1,2-dichloroethane, and prolonging the reaction time to 90 minutes. At the end there were metered 18.6 mmols of unreacted $H_2O_2$ (conversion: 85%) and 88 mmols of 1,2-epoxyoctane (selectivity: 83.5% on the $H_2O_2$ consumed).

Example 15

There was followed the procedure described in Example 1, but substituting the methyltrioctylammonium chloride with 1.25 g of triphenylmethylphosphonium chloride (4 mmoles) dissolved in 40 ml of methylene chloride.

By evaporation of the organic phase (preliminary filtered on paper) at 45—50°C under vacuum, there was obtained a white solid which, according to a percentual analysis, was found to be of the formula:

$$C_{57}H_{54}P_4W_4O_{24}=[(C_6H_5)_3PCH_3]_3PW_4O_{24}$$

| Elementary analysis | Theoretical % | Found % |
|---|---|---|
| C | 34.53 | 34.49 |
| H | 2.75 | 2.90 |
| P | 6.25 | 6.40 |
| W | 37.10 | 36.80 |

Active [0] found: 6.33%; active [0] theoretical (calculated for 8 $O_{act}$)=6.46%. Molecular weight (in 1,2-dichloroethane)=2120 (theoretical=1982.28).

Example 16

There was followed the procedure described in Example 9, but using the composition of Example 5 (1.4 g, corresponding to 2.5 mmols of W) instead of the composition of Example 1, and reducing the reaction time to 35 minutes. At the end there were metered 0.75 mmols of unreacted $H_2O_2$ (conversion: 99.4%) and 99.0 mmols of 1,2-epoxyoctane (selectivity: 80.3% on the $H_2O_2$).

Example 17

There was followed the procedure described in Example 9, but instead of the composition of Example 1 there was used the catalyst supported on a polystyrenic matrix of Example 8 (9 g of resin, corresponding to about 8 mmols of W), and by prolonging the reaction time to 2 hours. At the end there were metered 1.74 mmols of unreacted $H_2O_2$ (conversion: 98.6%) and 97.8 mmols of 1,2-epoxyoctane (selectivity: 80% on the $H_2O_2$).

Example 18

To 11.3 g (corr. to about 5.1 mmols) of the composition of Example 1, dissolved in 35 ml of benzene, there were added 25 ml of cyclohexene (about 200 mmols). This mixture, kept under stirring, was brought up to 70°C and maintained at this temperature for 1 hour. At the end there were metered by gas chromatography 1.93 g (19.7 mmols) of epoxycyclohexane.

Example 19

There was followed the procedure described in Example 18, but using 1-octene (31 ml; about 200 mmols) instead of cyclohexene. At the end there were metered by gas chromatography 2.93 g (22.9 mmols) of 1,2-epoxyoctane.

**Claims**

1. A peroxidic composition, characterized by substantially consisting of a peroxy-phospho-tungstate and/or a peroxy-arseno-tungstate having the following formula (I):

$$Q_3XW_4O_{24-2n} \quad (I)$$

wherein X represents a P or As atom; *n* is 0, 1 or 2; and Q represents a cation of a quaternary onium salt having the formula (II):

$$(R_1, R_2, R_3, R_4\ M)^+Y^- \quad (II)$$

wherein M represents a pentavalent element of Group VA of the Periodic System; $Y^-$ represents an inorganic anion; and $R_1$, $R_2$, $R_3$ and $R_4$, which may be the same as or different from each other, each represent a hydrogen atom or a monovalent hydrocarbon group having up to a total of 70 carbon atoms.

2. A peroxidic composition as claimed in claim 1, characterized in that $Y^-$ is $Cl^-$, $HSO_4^-$ or $NO_3^-$.

3. A peroxidic composition as claimed in claim 1 or 2, characterized in that $R_1$, $R_2$, $R_3$ and $R_4$ have a total of from 25 to 40 carbon atoms.

4. A peroxidic composition of the formula:

$$C_{75}H_{162}N_3PW_4O_{22}$$

or

$$[(C_8H_{17})_3NCH_3]_3PW_4O_{22}.$$

5. A peroxidic composition of the formula:

$$C_{72}H_{156}N_3PW_4O_{22}$$

or

$$[(C_6H_{13})_4N]_3PW_4O_{22}.$$

6. A peroxidic composition of the formula:

$$C_{72}H_{156}N_3AsW_4O_{24}$$

or

$$[(C_6H_{13})_4N]_3AsW_4O_{24}.$$

7. A peroxidic composition of the formula:

$$C_{48}H_{108}N_3PW_4O_{24}$$

or

$$[(C_4H_9)_4N]_3PW_4O_{24}.$$

8. A peroxidic composition of the formula:

$$C_{75}H_{162}N_3AsW_4O_{20}$$

or

$$[(C_8H_{17})_3NCH_3]_3AsW_4O_{20}.$$

9. A peroxidic composition of the formula:

$$C_{48}H_{108}N_3AsW_4O_{24}$$

or

$$[(C_4H_9)_4N]_3AsW_4O_{24}.$$

10. A peroxidic composition of the formula:

$$C_{111}H_{234}N_3PW_4O_{22}$$

or

$$[[C_{18}H_{37}(75\%)+C_{16}H_{33}(25\%)]_2N(CH_3)_2]_3PW_4O_{22}.$$

11. A peroxidic composition of the formula:

$$C_{57}H_{54}P_4W_4O_{24}$$

or

$$[(C_6H_5)_3PCH_3]_3PW_4O_{24}.$$

12. A process for the preparation of a peroxidic composition of formula (I) as defined in claim 1, characterized in that an oxygenated derivative of tungsten and an oxygenated derivative of P or As and hydrogen peroxide, contained in an acid aqueous phase, are reacted with a quaternary onium salt or with a precursor thereof, wherein the quaternary onium salt is of formula (II):

$$(R_1,\ R_2,\ R_3,\ R_4M)^+Y^- \qquad (II)$$

wherein M represents a pentavalent element of Group VA of the Periodic System; $Y^-$ represents an inorganic anion; and $R_1$, $R_2$, $R_3$ and $R_4$, which may be the same as or different from each other, each represent a hydrogen atom or a monovalent hydrocarbon group having up to a total of 70 carbon atoms, and in that the pH value in the aqueous phase is maintained below 2.

13. A process as claimed in claim 12, characterized in that the reaction is carried out at a temperature from 20°C to 80°C.

14. A process as claimed in claim 12 or 13, characterized in that there is used an oxygenated hexavalent tungsten compound selected from tungstic acid and its alkaline salts.

15. A process as claimed in claim 14, characterized in that the oxygenated hexavalent tungsten derivative is prepared *in situ*, under the conditions of the reaction, starting from a tungsten derivative selected from $WO_2$, $W_2O_5$, $WO_3$, $WS_2$, $WS_3$, W oxychloride, W chloride, W-hexacarbonyl or starting from metallic tungsten.

16. A process as claimed in any of claims 12 to 15, characterized in that there is used an oxygenated compound of pentavalent P selected from phosphoric acid and its alkaline salts.

17. A process as claimed in claim 16, characterized in that the oxygenated derivative of pentavalent P is prepared *in situ*, under the conditions of the reaction, starting from a P derivative selected from $P_2O_5$, $PCl_5$, $POCl_3$, polyphosphoric acid and its alkaline salts.

18. A process as claimed in any of claims 12 to 15, characterized in that there is used an oxygenated compound of pentavalent As selected from arsenic acid and its alkaline salts.

19. A process as claimed in claim 18, characterized in that the oxygenated derivative of pentavalent As is prepared *in situ*, under the conditions of the reaction, starting from a derivative selected from $As_2O_5$, $AsCl_5$ and $AsOCl_3$.

20. A process as claimed in any of claims 12 to 19, characterized in that $Y^-$ is $Cl^-$, $HSO_4^-$ or $NO_3^-$ in formula (II).

21. A process as claimed in any of claims 12 to 20, characterized in that $R_1$, $R_2$, $R_3$ and $R_4$ in the formula (II) have in total from 25 to 40 carbon atoms.

22. A process as claimed in any of claims 12 to 21, characterized in that, for M=N in the formula (II), the quaternary ammonium salt is prepared *in situ* under the conditions of the reaction, starting from the equivalent amine.

23. A process as claimed in any of claims 12 to 22, characterized in that the pH value in the aqueous phase is maintained below 2 by the addition of a mineral acid.

24. A process as claimed in any of claims 12 to 23, characterized in that the quaternary onium salt or its precursor is used dissolved in a substantially water-immiscible organic phase.

25. A process as claimed in claim 24, characterized in that the organic phase substantially immiscible with the aqueous phase consists of a solvent selected from aromatic and chlorinated hydrocarbons and mixtures thereof.

26. A process as claimed in claim 25, characterized in that the said organic solvent is selected from benzene, toluene, xylenes, dichloromethane, dichloroethane, trichloroethane, chlorobenzene and mixtures thereof.

27. A process as claimed in any of claims 12 to 26, characterized in that for each mol of the P or As compound, expressed as P or As, there are used at least 4 mols of the tungsten compound, expressed as W, and up to 2 mols of quaternary onium salt, and in that there are used from 2.5 to 6 mols of $H_2O_2$ for each mol of W (VI) compound.

28. A process as claimed in claim 27, characterized in that there is used an excess of $H_2O_2$ for the oxidation of W compound having a valence lower than VI.

29. A process for the preparation of an epoxidation catalyst fixed on a polymeric macroporous polystyrenic or silicone resin, characterized in that an oxygenated tungsten derivative, and an oxygenated derivative of P or As and hydrogen peroxide, contained in an aqueous phase having a pH below 2, are reacted with an onium salt fixed on a polymeric macroporous polystyrenic or silicone resin.

**Patentansprüche**

1. Eine Peroxidzusammensetzung, dadurch gekennzeichnet, daß sie im wesentlichen aus einem Peroxy-phosphor-wolframat und/oder einem Peroxy-arseno-wolframat mit der folgenden Formel (I)

$$Q_3XW_4O_{24-2n} \qquad (I)$$

worin X für ein P oder As Atom steht, n=0, 1 oder 2 ist und Q ein Kation eines quaternären Onium-salzes mit der Formel (II) darstellt

$$(R_1 . R_2 . R_3 . R_4M)^+Y^- \qquad (II)$$

worin M ein 5-wertiges Element aus der Gruppe VA des Periodensystems bedeutet; $Y^-$ ein anorganisches Anion bedeutet und $R_1$, $R_2$, $R_3$ und $R_4$, die gleich oder voneinander verschieden sein können, jeweils für ein Wasserstoffatom oder eine einwertige Kohlenwasserstoffgruppe mit bis zu insgesamt 70 Kohlenstoffatomen stehen, besteht.

2. Peroxidzusammensetzung nach Anspruch 1, dadurch gekennzeichnet, daß $Y^-$ für $Cl^-$, $HSO_4^-$ oder $NO_3^-$ steht.

3. Peroxidzusammensetzung nach Anspruch 1 oder 2, dadurch gekennzeichnet, daß $R_1$, $R_2$, $R_3$ und $R_4$ insgesamt von 25 bis 40 Kohlenstoffatome haben.

4. Peroxidzusammensetzung mit der Formel

$$C_{75}H_{162}N_3PW_4O_{22}$$

oder

$$[(C_8H_{17})_3NCH_3]_3PW_4O_{22}.$$

5. Peroxidzusammensetzung mit der Formel

$$C_{72}H_{156}N_3PW_4O_{22}$$

oder

$$[(C_6H_{13})_4N]_3PW_4O_{22}.$$

6. Peroxidzusammensetzung mit der Formel

$$C_{72}H_{156}N_3AsW_4O_{24}$$

oder

$$[(C_6H_{13})_4N]_3AsW_4O_{24}.$$

7. Peroxidzusammensetzung mit der Formel

$$C_{48}H_{108}N_3PW_4O_{24}$$

oder

$$[(C_4H_9)_4N]_3PW_4O_{24}.$$

8. Peroxidzusammensetzung mit der Formel

$$C_{75}H_{162}N_3AsW_4O_{20}$$

oder

$$[(C_8H_{17})_3NCH_3]_3AsW_4O_{20}.$$

9. Peroxidzusammensetzung mit der Formel

$$C_{48}H_{108}N_3AsW_4O_{24}$$

oder

$$[(C_4H_9)_4N]_3AsW_4O_{24}.$$

10. Peroxidzusammensetzung mit der Formel

$$C_{111}H_{234}N_3PW_4O_{22}$$

oder

$$[[(C_{18}H_{37}(75\%)+C_{16}H_{33}(25\%)]_2N(CH_3)_2]_3PW_4O_{22}.$$

11. Peroxidzusammensetzung mit der Formel

$$C_{57}H_{54}P_4W_4O_{24}$$

oder

$$[(C_6H_5)_3PCH_3]_3PW_4O_{24}.$$

12. Ein Verfahren zur Herstellung einer Peroxidzusammensetzung der Formel (I) gemäß Definition in Anspruch 1, dadurch gekennzeichnet, daß ein sauerstoffhaltiges Derivat von Wolfram oder ein sauerstoffhaltiges Derivat von P oder As und Wasserstoffperoxid, enthalten in einer sauren wässrigen Phase, mit einem quarternären Onium-salz oder mit einem Vorläufer desselben umgesetzt werden, wobei das quaternäre Onium-salz die Formel (II) hat

$$(R_1 . R_2 . R_3 . R_4M)^+Y^- \qquad (II)$$

worin M ein 5-wertiges Element der Gruppe VA des Periodensystems bedeutet; $Y^-$ ein anorganisches Anion bedeutet, und $R_1$, $R_2$, $R_3$ und $R_4$, die gleich oder voneinander verschieden sein können, jeweils in Wasserstoffatom oder eine einwertige Kohlenwasserstoffgruppe mit bis zu insgesamt 70 Kohlenstoffatomen bedeuteten, und daß der pH-Wert in der wässrigen Phase unterhalb 2 gehalten wird.

13. Ein Verfahren nach Anspruch 12, dadurch gekennzeichnet, daß die Reaktion bei einer Temperatur von 20 bis 80°C durchgeführt wird.

14. Ein Verfahren nach Anspruch 12 oder 13, dadurch gekennzeichnet, daß man eine sauerstoffhaltige 6-wertige Wolframverbindung, ausgewählt aus Wolframsäure und deren Alkalisalzen, verwendet.

15. Ein Verfahren nach Anspruch 14, dadurch gekennzeichnet, daß das sauerstoffhaltige 6-wertige Wolframderivat in situ unter den Bedingungen der Reaktion, ausgehend von einem Wolframderivat, ausgewählt aus $WO_2$, $W_2O_5$, $WO_3$, $WS_2$, $WS_3$, W-Oxychlorid, W-Chlorid, W-Hexacarbonyl, oder ausgehend von metallischem Wolfram hergestellt wird.

16. Ein Verfahren nach irgendeinem der Ansprüche 12 bis 15, dadurch gekennzeichnet, daß man eine sauerstoffhaltige Verbindung des 5-wertigen P, ausgewählt aus Phosphorsäure und deren Alkalisalzen, verwendet.

17. Ein Verfahren nach Anspruch 16, dadurch gekennzeichnet, daß das sauerstoffhaltige Derivat des 5-wertigen P in situ unter den Bedingungen der Reaktion, ausgehend von einem P-Derivat, ausgewählt aus $P_2O_5$, $PCl_5$, $POCl_3$, Polyphosphorsäure und deren Alkalisalzen, hergestellt wird.

18. Ein Verfahren nach irgendeinem der Ansprüche 12 bis 15, dadurch gekennzeichnet, daß man eine sauerstoffhaltige Verbindung des 5-wertigen As, ausgewählt aus Arsensäure und deren Alkalisalzen, verwendet.

19. Ein Verfahren nach Anspruch 18, dadurch gekennzeichnet, daß das sauerstoffhaltige Derivat des 5-wertigen As in situ unter den Bedingungen der Reaktion, ausgehend von einem Derivat, ausgewählt aus $As_2O_5$, $AsCl_5$ und $AsOCl_3$, hergestellt wird.

20. Ein Verfahren nach irgendeinem der Ansprüche 12 bis 19, dadurch gekennzeichnet, daß $Y^-$ für $Cl^-$, $HSO_4^-$ oder $NO_3^-$ in Formel (II) steht.

21. Ein Verfahren nach irgendeinem der Ansprüche 12 bis 20, dadurch gekennzeichnet, daß $R_1$, $R_2$, $R_3$ und $R_4$ in Formel (II) insgesamt von 25 bis 40 Kohlenstoffatome besitzen.

22. Ein Verfahren nach irgendeinem der Ansprüche 12 bis 21, dadurch gekennzeichnet, daß das quarternäre Ammoniumsalz in situ unter den Bedingungen der Reaktion, ausgehend vom Aminäquivalent hergestellt wird, wenn in Formel (II) M=N ist.

23. Ein Verfahren nach irgendeinem der Ansprüche 12 bis 22, dadurch gekennzeichnet, daß der pH-Wert in der wässrigen Phase durch Zugabe einer Mineralsäure unter 2 gehalten wird.

24. Ein Verfahren nach irgendeinem der Ansprüche 12 bis 23, dadurch gekennzeichnet, daß das quarternäre Onium-salz oder dessen Vorläufer, in einer praktisch mit Wasser nicht mischbaren, organischen Phase gelöst, verwendet wird.

25. Ein Verfahren nach Anspruch 24, dadurch gekennzeichnet, daß die organische, mit der wässrigen Phase praktisch nicht mischbare Phase aus einem Lösungsmittel, ausgewählt aus aromatischen und chlorierten Kohlenwasserstoffen und Mischungen derselben, besteht.

26. Ein Verfahren nach Anspruch 25, dadurch gekennzeichnet, daß das organische Lösungsmittel aus Benzol, Toluol, Xylolen, Dichlormethan, Dichlorethan, Trichloethan, Chlorbenzol und Mischungen derselben ausgewählt ist.

27. Ein Verfahren nach irgendeinem der Ansprüche 12 bis 26, dadurch gekennzeichnet, daß man pro

Mol der P- oder As-Verbindung, ausgedrückt als P oder As, mindestens 4 Mol der Wolframverbindung, ausgedrückt als W, und bis zu 2 Mol des quarternären Onium-salzes verwendet und daß von 2,5 bis 6 Mol $H_2C_2$ pro Mol der W(VI)-Verbindung verwendet werden.

28. Ein Verfahren nach Anspruch 27, dadurch gekennzeichnet, daß zur Oxidation der W-Verbindung mit einer Wertigkeit unter VI ein Überschuß an $H_2O_2$ verwendet wird.

29. Ein Verfahren zur Herstellung eines auf einem polymeren makroporösen Polystyrol-artigen oder Silicon-Harz fixierten Epoxidationskatalysators, dadurch gekennzeichnet, daß ein sauerstoffhaltiges Wolframderivat und ein sauerstoffhaltiges Derivat von P oder As und Wasserstoffperoxid, enthalten in einer wässrigen Phase mit einem pH unter 2, mit einem Onium-salz umgesetzt werden, das auf einem polymeren makroporösen Polystyrol-artigen oder Siliconharz fixiert ist.

**Revendications**

1. Une composition peroxydique caractérisé par le fait qu'elle consiste sensiblement en un peroxy-phospho-tungstate et/ou un peroxy-arceno-tungstate ayant la formule (I) suivante:

$$Q_3XW_4O_{24-2n} \qquad (I)$$

dans laquelle:

X représente un atome P ou As;

n est 0, 1 ou 2;

Q représente un cation d'un sel d'onium quaternaire ayant la formule (II):

$$(R_1,\ R_2,\ R_3,\ R_4,\ M)^+Y^- \qquad (II)$$

dans laquelle:

M représente un élément pentavalent du groupe VA du système périodique;

$Y^-$ représente un anion minéral; et

$R_1$, $R_2$, $R_3$ et $R_4$ qui peuvent être identiques ou différents les uns des autres représentent chacun un atome d'hydrogène ou un groupe hydrocarboné monovalent ayant jusqu'à un total de 70 atomes de carbone.

2. Une composition peroxydique telle que revendiquée à la revendication 1, caractérisée en ce que $Y^-$ est $Cl^-$, $HSO_4^-$ ou $NO_3^-$.

3. Une composition peroxydique telle que revendiquée à la revendication 1 ou 2, caractérisée en ce que $R_1$, $R_2$, $R_3$ et $R_4$ ayant un total de 25 à 40 atomes de carbone.

4. Une composition peroxydique de formule:

$$C_{75}H_{162}N_3PW_4O_{22}$$

ou

$$[(C_8H_{17})_3NCH_3]_3PW_4O_{22}.$$

5. Une composition peroxydique de formule:

$$C_{72}H_{156}N_3PW_4O_{22}$$

ou

$$[(C_6H_{13})_4N]_3PW_4O_{22}.$$

6. Une composition peroxydique de formule:

$$C_{72}H_{156}N_3AsW_4O_{24}$$

ou

$$[(C_6H_{13})_4N]_3AsW_4O_{24}.$$

7. Une composition peroxydique de formule:

$$C_{48}H_{108}N_3PW_4O_{24}$$

ou

$$(C_4H_9)_4N_3PW_4O_{24}.$$

8. Une composition peroxydique de formule:

$$C_{75}H_{162}N_3AsW_4O_{20}$$

ou

$$(C_8H_{17})_3NCH_{33}AsW_4O_{20}.$$

9. Une composition peroxydique de formule:

$$[C_{48}H_{108}N_3As]W_4O_{24}$$

ou

$$(C_4H_9)_4N_3AsW_4O_{24}.$$

10. Une composition peroxydique de formule:

$$[C_{111}H_{234}N]_3PW_4O_{22}$$

ou

$$(C_{18}H_{37}(75\%)+C_{16}H_{33}(25\%)_2N(CH_3)_{23}PW_4O_{22}.$$

11. Une composition peroxydique de formule:

$$\{[C_{57}H_{54}P_4W_4O_{24}]\}$$

ou

$$(C_6H_5)_3PCH_{33}PW_4O_{24}.$$

12. Un procédé pour la préparation d'une composition peroxydique de formule (I) telle que définie à la revendication 1, caractérisé en ce qu'un dérivé oxygéné du tungstène et un dérivé oxygéné de P ou As et du peroxyde d'hydrogène, contenus dans une phase aqueuse acide réagissent avec un sel d'onium quaternaire ou avec un de ses précurseurs, dans lequel le sel d'onium quaternaire est de formule (II):

$$(R_1, R_2, R_3, R_4, M)^+Y^- \quad (II)$$

dans laquelle:

M représente un élément pentavalent du groupe VA du système périodique;

$Y^-$ représente un anion minéral; et

$R_1$, $R_2$, $R_3$ et $R_4$ qui peuvent être identiques ou différents les uns des autres représentent chacun un atome d'hydrogène ou un groupe hydrocarboné monovalent ayant jusqu'à un total de 70 atomes de carbone, et en ce que la valeur du pH dans la phase aqueuse est maintenue en-dessous de 2.

13. Un procédé tel que revendiqué à la revendication 12, caractérisé en ce que la réaction est menée à une température de 20°C à 80°C.

14. Un procédé tel que revendiqué à la revendication 12 ou 13, caractérisé en ce que l'on utilise un composé de tungstène hexavalent oxygéné choisi parmi l'acide tungstique et ses sels alcalins.

15. Un procédé tel que revendiqué à la revendication 14, caractérisé en ce que le dérivé de tungstène hexavalent oxygéné et préparé *in situ*, sous les conditions de réaction partant d'un dérivé du tungstène choisi parmi $WO_2$, $W_2O_5$, $WO_3$, $WS_2$, $WS_3$, oxychlorure de W, chlorure de W, W hexacarbonyle ou partant de tungstène métal.

16. Un procédé tel que revendiqué selon l'une quelconque des revendications 12 à 15, caractérisé en ce qu'on utilise un composé oxygéné de P pentavalent choisi parmi l'acide phosphorique et ses sels alcalins.

17. Un procédé tel que revendiqué à la revendication 16, caractérisé en ce que le dérivé oxygéné de P pentavalent est préparé *in situ* sous les conditions de réaction, en partant d'un dérivé de P choisi parmi $P_2O_5$, $PCl_5$, $POCl_3$, l'acide polyphosphorique et ses sels alcalins.

18. Un procédé tel que revendiqué dans l'une quelconque des revendications 12 à 15, caractérisé en ce que l'on utilise un composé oxygéné de l'As pentavalent choisi parmi l'acide arsénique et ses sels alcalins.

19. Un procédé tel que revendiqué à la revendication 18, caractérisé en ce que le dérivé oxygéné de l'As pentavalent est préparé in situ, sous les conditions de la réaction, en partant d'un dérivé choisi parmi $As_2O_5$, $AsCl_5$ et $AsOCl_3$.

20. Un procédé tel que revendiqué selon l'une quelconque des revendications 12 à 19, caractérisé en ce que $Y^-$ est $Cl^-$, $HSO_4^-$ ou $NO_3^-$ dans la formule (II).

21. Un procédé tel que revendiqué dans l'une quelconque des revendications 12 à 20, caractérisé en ce que $R_1$, $R_2$, $R_3$ et $R_4$ dans la formule (II) ont, au total, de 25 à 40 atomes de carbone.

22. Un procédé tel que revendiqué dans l'une quelconque des revendications 12 à 21, caractérisé en ce que pour M=N dans la formule (II), le sel d'ammonium quaternaire est préparé *in situ*, sous les conditions de la réaction, en partant de l'amine équivalente.

23. Un procédé tel que revendiqué dans l'une quelconque des revendications 12 à 22, caractérisé en ce que la valeur du pH dans la phase aqueuse est maintenue en-dessous de 2 par addition d'un acide minéral.

24. Un procédé tel que revendiqué selon l'une quelconque des revendications 12 à 23, caractérisé en ce que le sel d'onium quaternaire ou l'un de ses précurseurs est utilisé à l'état dissous dans une phase organique sensiblement non miscible à l'eau.

25. Un procédé tel que revendiqué à la revendication 24, caractérisé en ce que la phase organique sensiblement non miscible avec la phase aqueuse consiste en un solvant choisi parmi les hydrocarbures aromatiques et chlorés ou leurs mélanges.

26. Un procédé tel que revendiqué à la revendication 25, caractérisé en ce que ledit solvant organique est choisi parmi le benzène, le toluène, les xylènes, le dichlorométhane, le dichloroéthane, le trichloroéthane, le chlorobenzène et leurs mélanges.

27. Un procédé tel que revendiqué dans l'une quelconque des revendications 12 à 26, caractérisé en ce que pour chaque molécule du composé de P ou de As exprimée en P ou en As, on utilise au moins quatre molécules du composé de tungstène exprimées en W, et jusqu'à deux molécules de sel d'onium quaternaire, et en ce que l'on utilise de 2,5 à 6 molécules de $H_2O_2$ pour chaque molécule de composé de W (IV).

28. Un procédé tel que revendiqué à la revendication 27, caractérisé en ce que l'on utilise un excès de $H_2O_2$ pour l'oxydation du composé de W ayant une valence inférieure à 6.

29. Un procédé pour la préparation d'un catalyseur d'époxydation fixé sur une résine polymère macroporeuse polystyrénique ou silicone, caractérisé en ce qu'un dérivé oxygéné de tungstène et un dérivé oxygéné de P ou As et du peroxyde d'hydrogène contenus dans une phase aqueuse ayant un pH en-dessous de 2 réagissent avec un sel d'onium fixé sur une résine polymère macroporeuse polystyrénique ou silicone.